# EUROPEAN PATENT SPECIFICATION

(11) **EP 2 041 083 B1**
(45) Date of publication and mention of the grant of the patent: **05.11.2014**
(21) Application number: 07733717.8
(22) Date of filing: 25.05.2007
(51) Int. Cl.: C07D 207/16, A61K 31/401, A61P 9/00

(54) **METHODS OF PREPARING ZOFENOPRIL CALCIUM**
VERFAHREN ZUR HERSTELLUNG VON ZOFENOPRIL CALZIUM
PROCÉDÉ DE PRÉPARATION DE ZOFÉNOPRIL CALCIUM

(30) Priority: 26.05.2006 IN MU08152006
(43) Date of publication of application: 01.04.2009
(73) Proprietor: Generics [UK] Limited, Potters Bar Hertfordshire EN6 1AG (GB)
(72) Inventor: GORE, Vinayak G., Maharashtra 410208 (IN); PEHERE, Ashok D., Maharashtra 410208 (IN); GAIKWAD, Avinash C., Maharashtra 410208 (IN); VIJAYKAR, Priyesh S., Maharashtra 410208 (IN)
(74) Representative: Elend, Almut Susanne
(86) International application number: PCT/GB2007/050296
(87) International publication number: WO 2007/138352

(56) References cited:
- WO-A-00/07984
- WO-A-2007/003963

## Description

### Field of the invention

The present invention relates to methods of preparing zofenopril calcium. Zofenopril calcium, (4S)-1-[(2S)-3-benzoylthio-2-methylpropionyl]-4-(phenylthio)-L-proline calcium salt, has the following structure:

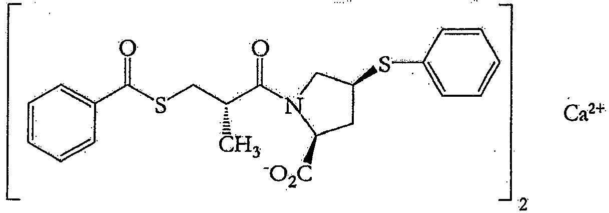

### Background of the invention

The manufacturing process for many pharmaceuticals is hindered by the fact that the organic compound which is the active drug substance is difficult to handle during the manufacturing process. These difficulties can lead to undesirable properties being imparted to the final drug or dosage form. In addition, it can be difficult to control the polymorphic form of the active drug substance throughout the manufacturing process.

For pharmaceuticals in which the active ingredient can exist in more than one polymorphic form, it is particularly important to ensure that the manufacturing process for the active ingredient affords a single polymorph with a consistent level of polymorphic purity. If the process leads to polymorph with varying degrees of polymorphic purity and/or where the process does not control polymorphic interconversion, serious problems with dissolution and/or bioavailability can arise in the finished pharmaceutical formulation comprising the active ingredient. Zofenopril calcium, (4S)-1-1(2S)-3-benzoylthio-2-methylproplonyl]-4-(phenylthio)-L-proline calcium salt, is a non-peptidic, orally active, long acting ACE inhibitor. It is marketed for the treatment of hypertension under the trade name Zoprace^{®}.

Zofenopril and methods for its preparation were first described in US patent US 4,316,906. Two polymorphs of zofenopril calcium, forms A and B, were disclosed in US patent US 6,515,012 and international patent application WO 00/07984. It was also disclosed in US 6,515,012 and WO 00/07984 that polymorph A is much more industrially suitable than form B. Polymorph forms A and B of zofenopril calcium disclosed in US 6,515,012 and WO 00/07984 show the following XRD patterns:

**Table 1 - XRD Comparison Position [26 values]**

| **Polymorph A** | **Polymorph B** |
|---|---|
| 4.3 | |
| | 4.8 |
| 7.4 | |
| 8.7 | |
| 10.1 | |
| 10.8 | |
| 11.7 | |
| 13.0 | |
| 14.8 | |
| | 15.6 |
| 16.0 | |
| 17.2 | |
| | 17.5 |
| 18.2 | |
| | 18.5 |
| 19.0 | |
| | 19.4 |
| 20.0 | |
| | 20.5 |
| | 21.4 |
| 21.7 | |
| | 21.8 |
| | 23.1 |
| 23.5 | |
| 24.6 | 24.6 |

One method for the preparation of zofenopril calcium as disclosed in US 4,316,906 mainly yields polymorph A, but there is always contamination with polymorph B to varying degrees (never below 20%). An alternative synthesis described in US 4,316,906 affords a partially amorphous product, with very variable characteristics, in which polymorph A, when present, is in concentrations much lower than those obtained in the preceding process.

A process for the preparation of supposedly pure form A is disclosed in US 6,515,012 and WO 00/07984, and is summarised below:
(a) reaction of S(-)-3-benzoylthio-2-methyl-propanoic acid chloride and cis-4-phenylthio-L-proline in water at pH ranging from 9.0 to 9.5 and recovery of zofenopril in the acidic form,
(b) salification of acid Zofenopril with a potassium salt in alcoholic solution and recovery of the resulting potassium salt,
(c) conversion of the potassium salt to calcium salt by addition of an aqueous solution of zofenopril potassium salt to a calcium chloride aqueous solution at up to 90°C with simultaneous seeding to promote the precipitation of polymorph A.

However, the above method for the preparation of polymorph A has the following drawbacks:
- The reaction is carried out at a relatively high temperature (up to 90°C) at which interconversion of the polymorphs is possible.
- Polymorph A can be obtained from the above process, but the product is always contaminated with polymorph B.
- The chemical HPLC purity of the zofenopril obtained is only between 97-98%.

Therefore there remains a need for an improved process for the preparation of zofenopril calcium form A with high polymorphic purity. There also remains a need for an improved process for the preparation of zofenopril calcium with high chemical purity.

### Object of the invention

It is an object of the present invention to provide a process for the preparation of zofenopril calcium polymorph A of high optical purity, in particular Zofenopril calcium form A which is substantially free of form B or other polymorphs. It is also an object of the present invention to provide a process for the preparation of zofenopril calcium of high chemical purity.

The process of the present invention may be used to provide Zofenopril calcium polymorph A of high optical purity. The process of the present invention may also be used to provide zofenopril calcium of high chemical purity.

Zofenopril calcium prepared by the process of the present invention may be used in compositions comprising zofenopril calcium polymorph A of high optical purity. The zofenopril calcium prepared by the process of the present invention may also be used in compositions comprising zofenopril calcium of high chemical purity.

The process of the present invention may be used to provide zofenopril calcium in a solid crystalline form that affords the compound improved handling properties (such as increased resistance to compression and/or micronisation) and/or improved properties as a pharmaceutical agent, and enables strict control of the purity and/or polymorphic form during manufacturing.

### Summary of the invention

The present invention provides a method of preparing zofenopril calcium, comprising the steps of:
a) combining an aqueous solution of a calcium salt and a solution of a zofenopril salt other than zofenopril calcium in an organic, water miscible solvent,
b) maintaining the resulting mixture until zofenopril calcium precipitates, and
c) separating the zofenopril calcium from the suspension.

The method of the present invention may be used to provide zofenopril calcium form A, substantially free of other forms of zofenopril calcium. Zofenopril calcium form A shows XRD peaks at substantially the following scattering angles 2θ: 4.3, 7.4, 8.7, 10.1, 10.8, 11.7, 13.0, 14.8, 16.0, 17.2, 18.2, 19.0, 20.0, 21.7, 23.5, and 24.6.

In the context of the present application, the term 'substantially free' of other forms of zofenopril calcium means that the zofenopril calcium form A comprises less than 6% of other crystalline or amorphous forms of Zofenopril calcium, preferably less than 5%, preferably less than 4%, preferably less than 3%, preferably less than 2%, preferably less than 1%.

The method of the present invention may also provide zofenopril calcium having a chemical purity of more than 98.5%, preferably more than 99%, preferably more than 99.3%, as measured by HPLC.

The zofenopril calcium prepared by the method of the present invention may be used in a pharmaceutical composition comprising zofenopril calcium.

The zofenopril calcium prepared according to the method of the present invention may be used for the manufacture of a medicament for the treatment of hypertension or another angiotensin-mediated condition.

The zofenopril calcium prepared by the method of the present invention may be used in a method of treating hypertension or another angiotensin-mediated condition, comprising administering a therapeutically effective amount of the zofenopril calcium to a patient in need thereof.

Preferably the method of the present invention comprises the use of zofenopril potassium or zofenopril dicyclohexylamine. Preferably the method comprises the use of calcium chloride. Preferably the method comprises the use of an organic solvent/water system.

The method of the present invention may comprise the step of reacting a zofenopril salt other than zofenopril calcium with an aqueous solution of a calcium salt in an organic solvent/water system.

As stated above, the present invention provides a method of preparing zofenopril calcium, comprising the steps of:
a) combining an aqueous solution of a calcium salt and a solution of a zofenopril salt other than zofenopril calcium in an organic, water miscible solvent,
b) maintaining the resulting mixture until zofenopril calcium precipitates, and
c) separating the zofenopril calcium from the suspension.

Preferably the calcium salt used in step (a) is soluble in water, preferably readily soluble in water. Calcium salts which are soluble in water include, but are not limited to, calcium chloride, calcium bromide, calcium iodide, calcium formate, calcium acetate, calcium propionate, calcium butyrate, calcium isobutyrate, calcium α-methyl butyrate, and calcium oxide. A preferred calcium salt used in step (a) is calcium chloride.

Preferably the zofenopril salt used in step (a) is a lithium, sodium, potassium, magnesium, amine or amino acid salt of zofenopril. Amine salts of zofenopril include, but are not limited to, the dibenzylamine, N,N-dibenzylethylenediamine, methylamine, dimethylamine, trimethylamine, ethylamine, diethylamine, triethylamine, N-ethylmethylamine, t-butylamine, N,N-diisopropylethylamine, N,N-diisopropylmethylamine, procaine, N-ethylpiperidine, cyclohexylamine, dicyclohexylamine, and 1-adamantanamine salt. Amino acid salts of zofenopril include, but are not limited to, the arginine and lysine salt. Preferred zofenopril salts used in step (a) are zofenopril potassium and zofenopril dicyclohexylamine.

Organic, water miscible solvents include, but are not limited to, alcohols, aliphatic ketones (such as acetone or ethyl methyl ketone), acetonitrile, formamide, N,N-dimethylformamide, dimethylsulfoxide, sulfolane, diglyme, dioxane, tetrahydrofuran, and N-methylpyrrolidine. Preferably the organic, water miscible solvent is a lower alkyl alcohol. Lower alkyl alcohols include, but are not limited to, methanol, ethanol, n-propanol, isopropanol and n-butanol. Preferably the organic, water miscible solvent is methanol.

In step (a), the aqueous solution of a calcium salt and the solution of a zofenopril salt in an organic, water miscible solvent are preferably combined in a water : organic solvent ratio of 1:1 to 1:10, preferably 1:1 to 1:5, more preferably 1:1 to 1:3.

Preferably the combining of step (a) is carried out at a temperature of up to 85°C, preferably at a temperature of 55-85°C, preferably 55-70°C, preferably 55-60°C.

Preferably the maintaining of step (b) is carried out with stirring and/or for at least one hour. Preferably the maintaining of step (b) is carried out at a temperature of up to 85°C, preferably at a temperature of 55-85°C, preferably 55-70°C, preferably 55-60°C.

Preferably the method is carried out without seeding zofenopril calcium form A.

The separating of step (c) may be achieved by filtering the suspension. Preferably the separating of step (c) is carried out at a temperature of up to 85°C, preferably up to 70°C, preferably up to 60°C, preferably up to 55°C.

The separated zofenopril calcium may be purified by washing with water, preferably until the zofenopril calcium is substantially free from chloride ions.

Alternatively or additionally, the separated zofenopril calcium may be purified by washing with methanol. The washing with methanol may be achieved by stirring the zofenopril calcium in methanol and filtering.

The separated zofenopril calcium may be dried under reduced pressure at a temperature of up to 85°C, preferably up to 70°C, preferably up to 60°C, preferably up to 50°C.

### Detailed description of the present invention

Zofenopril calcium polymorph A obtained by the prior art processes is always contaminated with polymorph B.

The method of the present invention may be used to provide zofenopril calcium form A substantially free of other polymorphs.

The method of the present invention may be used to provide polymorphs form A that has a level of polymorph form B or other polymorphs at a level of less than 10%, preferably less than 8%, more preferably less than 5%, even more preferably less than 3%, and most preferably less than 1%.

The method of the present invention may comprise the use of the dicyclohexylamine (DCHA) salt of the zofenopril acid.

The method of the present invention may comprise the use of the potassium salt of the zofenopril acid in an alcohol/water solvent system at relatively mild temperature conditions, which exercise strict control on polymorph composition thereby preventing the interconversion of polymorphs. The alcohol used can be any lower alkyl alcohol, but is preferably methanol.

Zofenopril calcium form A of very good HPLC (chemical) purity (more than 98.5%, preferably more than 99%, preferably more than 99.3%) may be prepared by the method of the present invention.

Zofenopril calcium prepared by the method of the present invention may be used in pharmaceutical compositions comprising pure polymorph A of zofenopril calcium.

The pharmaceutical compositions are preferably solid and can comprise a compound prepared in accordance with the method of the present invention in addition to one or more conventional pharmaceutically acceptable excipient(s). Preferred dosage forms include tablets, capsules and the like.

The compounds prepared by the method of the present invention may also be useful as precursors to other novel or known polymorphic forms of zofenopril that may be useful in the preparation of pharmaceutical products. Alternatively, the compounds prepared by the method of the present invention may be used to prepare other desired polymorphic forms of zofenopril in a more controllable manner.

The present invention is illustrated, but in no way limited, by the following examples.

### Example 1

An aqueous solution of calcium chloride was added to a methanolic solution (methanol : water = 1 : 1) of DCHA salt of zofenopril acid at 80-85°C and the resulting suspension was stirred for 3 hours. The resulting suspension was filtered off at 55°C and the product was washed with water until free from chloride ions. The wet cake was stirred in methanol and filtered and the wet solids were dried under reduced pressure at 50°C until a constant weight was obtained.
Purity = 99.4% (HPLC).
Melting point = 235-246°C; DSC endotherm at 257°C.
The XRPD pattern of the product thus obtained matched with the reported polymorph A (WO 00/07984).
The product obtained had only traces of form B detectable by XRPD, The detection limit of the XRPD instrument used was 3%.

### Example 2

An aqueous solution of calcium chloride was added to a methanolic solution (methanol : water = 3 : 1) of DCHA salt of zofenopril acid at 80-85°C and the resulting suspension was stirred for 3 hours. The resulting suspension was filtered off at 55°C and the product was washed with water until free from chloride ions. The wet cake was stirred in methanol and filtered and the wet solids were dried under reduced pressure at 50°C until a constant weight was obtained.
Purity = 99.39% (HPLC).
Melting point = 235-246°C; DSC endotherm at 254°C.
The XRD pattern of the product thus obtained matched with the reported polymorphs A (WO 00/07984).
The product obtained had only traces of form B detectable by XRPD. The detection limit of the XRPD instrument used was 3%.

### Example 3

An aqueous solution of calcium chloride was added to a methanolic solution (methanol : water = 1 : 1) of DCHA salt of zofenopril acid at 55-60°C and the resulting suspension was stirred for 3 hours. The resulting suspension was filtered off at 55°C and the product was washed with water until free from chloride ions. The wet cake was stirred in methanol and filtered and the wet solids were dried under reduced pressure at 50°C until a constant weight was obtained.
Purity = 99.24% (HPLC).
Melting point = 235-246°C; DSC endotherm at 255.7°C.
The XRPD pattern of the product thus obtained matched with the reported polymorph A (WO 00/07984).
The product obtained had only traces of form B detectable by XRPD. The detection limit of the XRPD instrument used was 3%.

### Example 4

An aqueous solution of calcium chloride was added to a methanolic solution (methanol : water = 1 : 1) of the potassium salt of zofenopril acid at 55-60°C and the resulting suspension was stirred for 3 hours. The resulting suspension was filtered off at 55°C and the product was washed with water until free from chloride ions. The wet cake was stirred in methanol and filtered and the wet solids were dried under reduced pressure at 50°C until a constant weight was obtained.
Purity = 99.43% (HPLC).
Melting point = 235-246°C; DSC endotherm at 258°C.
The XRPD pattern of the product thus obtained matched with the reported polymorph A (WO 00/07984)
The product obtained had no trace of form B detectable by XRPD. The detection limit of the XRPD instrument used was 3%.

## Claims

1. A method of preparing zofenopril calcium, comprising the steps of:
(a) combining an aqueous solution of a calcium salt and a solution of a zofenopril salt other than zofenopril calcium in an organic, water miscible solvent,
(b) maintaining the resulting mixture until zofenopril calcium precipitates, and
(c) separating the zofenopril calcium from the suspension.

2. A method as claimed in claim 1, wherein the calcium salt used in step (a) is:
(i) soluble in water; and/or
(ii) calcium chloride, calcium bromide, calcium iodide, calcium formate, calcium acetate, calcium propionate, calcium butyrate, calcium isobutyrate, calcium α-methyl butyrate, or calcium oxide; and/or
(iii) calcium chloride.

3. A method as claimed in claim 1 or 2, wherein the zofenopril salt used in step (a) is:
(i) a lithium, sodium, potassium, magnesium, amine or amino acid salt of zofenopril; and/or
(ii) zofenopril potassium or zofenopril dicyclohexylamine.

4. A method as claimed in any one of claims 1 to 3, wherein the organic, water miscible solvent is:
(i) an alcohol, an aliphatic ketone, acetonitrile, formamide, N,N-dimethylformamide, dimethylsulfoxide, sulfolane, diglyme, dioxane, tetrahydrofuran, or N-methylpyrrolidine; and/or
(ii) a lower alkyl alcohol; and/or
(iii) methanol.

5. A method as claimed in any one of claims 1 to 4, wherein:
(i) in step (a) the aqueous solution of a calcium salt and the solution of a zofenopril salt in an organic, water miscible solvent are combined in a water : organic solvent ratio of 1:1 to 1:10; and/or
(ii) the combining of step (a) is carried out at a temperature of up to 85°C; and/or
(iii) the maintaining of step (b) is carried out with stirring; and/or
(iv) the maintaining of step (b) is carried out for at least one hour; and/or
(v) the maintaining of step (b) is carried out at a temperature of up to 85°C; and/or
(vi) the method is carried out without seeding zofenopril calcium form A; and/or
(vii) the separating of step (c) is achieved by filtering the suspension; and/or
(viii) the separating of step (c) is carried out at a temperature of up to 85°C; and/or
(ix) the separated zofenopril calcium is purified by washing with water or methanol; and/or
(x) the separated zofenopril calcium is purified by washing with methanol by stirring the zofenopril calcium in methanol and filtering; and/or
(xi) the separated zofenopril calcium is dried under reduced pressure at a temperature of up to 85°C.

6. A method as claimed in any one of claims 1 to 5, wherein the zofenopril calcium prepared is zofenopril calcium form A.

7. A method as claimed in claim 6, wherein the zofenopril calcium form A is substantially free of other forms of zofenopril calcium.

8. A method as claimed in claim 6 or 7, wherein the zofenopril calcium form A shows XRD peaks at substantially the following scattering angles 2θ: 4.3, 7.4, 8.7, 10.1, 10.8, 11.7, 13.0, 14.8, 16.0, 17.2, 18.2, 19.0, 20.0, 21.7, 23.5, and 24.6.

9. A method as claimed in any one of claims 6 to 8, wherein the zofenopril calcium form A comprises less than 6% of other forms of zofenopril calcium.

10. A method as claimed in any one of claims 1 to 9, wherein the zofenopril calcium prepared has a chemical purity of more than 98.5%.

## Patentansprüche

1. Verfahren zum Herstellen von Zofenopril-Calciumsalz, welches folgende Schritte umfasst:
(a) Mischen einer wässrigen Lösung eines Calciumsalzes und einer Lösung eines Salzes von Zofenopril außer Zofenopril-Calciumsalz in einem organischen, mit Wasser mischbaren Lösungsmittel,
(b) Aufrechterhalten der sich ergebenden Mischung, bis Zofenopril-Calciumsalz ausfällt, und
(c) Abscheiden des Zofenopril-Calciumsalzes von der Suspension.

2. Verfahren nach Anspruch 1, wobei das in Schritt (a) verwendete Calciumsalz:
(i) in Wasser löslich ist; und/oder
(ii) Calciumchlorid, Calciumbromid, Calciumiodid, Calciumformiat, Calciumacetat, Calciumpropionat, Calciumbutyrat, Calciumisobutyrat, Calcium-α-methylbutyrat oder Calciumoxid ist; und/oder
(iii) Calciumchlorid ist.

3. Verfahren nach Anspruch 1 oder 2, wobei das in Schritt (a) verwendete Salz von Zofenopril:
(i) ein Lithium-, Natrium-, Kalium-, Magnesium-, Amin- oder Aminosäuresalz von Zofenopril ist; und/oder
(ii) Zofenopril-Kaliumsalz oder Zofenopril-Dicyclohexylaminsalz ist.

4. Verfahren nach einem der Ansprüche 1 bis 3, wobei das organische, in Wasser mischbare Lösungsmittel:
(i) ein Alkohol, ein aliphatisches Keton, Acetonitril, Formamid, N,N-Dimethylformamid, Dimethylsulfoxid, Sulfolan, Diglyme, Dioxan, Tetrahydrofuran oder N-Methylpyrrolidin ist; und/oder
(ii) ein Niederalkylalkohol ist; und/oder
(iii) Methanol ist.

5. Verfahren nach einem der Ansprüche 1 bis 4, wobei:
(i) in Schritt (a) die wässrige Lösung eines Calciumsalzes und die Lösung eines Salzes von Zofenopril in einem organischen, mit Wasser mischbarem Lösungsmittel in einem Verhältnis von Wasser zu organischem Lösungsmittel vermischt werden, welches zwischen 1:1 und 1:10 liegt; und/oder
(ii) das Mischen in Schritt (a) bei einer Temperatur von bis zu 85 °C ausgeführt wird; und/oder
(iii) das Aufrechterhalten in Schritt (b) unter Rühren ausgeführt wird; und/oder
(iv) das Aufrechterhalten in Schritt (b) wenigstens eine Stunde lang ausgeführt wird; und/oder
(v) das Aufrechterhalten in Schritt (b) bei einer Temperatur von bis zu 85 °C ausgeführt wird; und/oder
(vi) das Verfahren ohne Einimpfen von des Polymorphs A von Zofenopril-Calciumsalz ausgeführt wird; und/oder
(vii) das Abscheiden in Schritt (c) durch Filtern der Suspension erreicht wird; und/oder
(viii) das Abscheiden in Schritt (c) bei einer Temperatur von bis zu 85 °C ausgeführt wird; und/oder
(ix) das abgeschiedene Zofenopril-Calciumsalz durch Waschen mit Wasser oder Methanol gereinigt wird; und/oder
(x) das abgeschiedene Zofenopril-Calciumsalz durch Waschen mit Methanol gereinigt wird, indem das Zofenopril-Calciumsalz in Methanol eingerührt und gefiltert wird; und/oder
(xi) das abgeschiedene Zofenopril-Calciumsalz unter verringertem Druck bei einer Temperatur von bis zu 85 °C getrocknet wird.

6. Verfahren nach einem der Ansprüche 1 bis 5, wobei das Zofenopril-Calciumsalz der Polymorph A von Zofenopril-Calciumsalz ist.

7. Verfahren nach Anspruch 6, wobei der Polymorph A von Zofenopril-Calciumsalz im Wesentlichen frei von anderen Polymorphen von Zofenopril-Calciumsalz ist.

8. Verfahren nach Anspruch 6 oder 7, wobei der Polymorph A von Zofenopril-Calciumsalz in der Kristallstrukturanalyse Spitzen aufweist, die im Wesentlichen den folgenden Streuwinkeln entsprechen 2θ: 4,3, 7,4, 8,7, 10,1, 10,8, 11,7, 13,0, 14,8, 16,0, 17,2, 18,2, 19,0, 20,0, 21,7, 23,5 und 24,6.

9. Verfahren nach einem der Ansprüche 6 bis 8, wobei der Polymorph A von Zofenopril-Calciumsalz weniger als 6 % anderer Polymorphe von Zofenopril-Calciumsalz aufweist.

10. Verfahren nach einem der Ansprüche 1 bis 9, wobei das hergestellte Zofenopril-Calciumsalz eine chemische Reinheit von über 98,5 % aufweist.

## Revendications

1. Procédé de préparation de zofénopril calcium, comprenant les étapes consistant à :
(a) mélanger une solution aqueuse d'un sel de calcium et une solution d'un sel de zofénopril autre que le zofénopril calcium dans un solvant organique miscible à l'eau,
(b) conserver le mélange obtenu jusqu'à ce que le zofénopril calcium précipite, et
(c) séparer le zofénopril calcium de la suspension.

2. Procédé selon la revendication 1, dans lequel le sel de calcium utilisé à l'étape (a) est :
(i) soluble dans l'eau ; et/ou
(ii) du chlorure de calcium, du bromure de calcium, de l'iodure de calcium, du formiate de calcium, de l'acétate de calcium, du propionate de calcium, du butyrate de calcium, de l'isobutyrate de calcium, de l'α-méthylbutyrate de calcium ou de l'oxyde de calcium ; et/ou
(iii) du chlorure de calcium.

3. Procédé selon la revendication 1 ou 2, dans lequel le sel de zofénopril utilisé à l'étape (a) est :
(i) un sel de lithium, de sodium, de potassium, de magnésium, d'amine ou d'acide aminé de zofénopril ; et/ou
(ii) du zofénopril potassium ou de la zofénopril dicyclohexylamine.

4. Procédé selon l'une quelconque des revendications 1 à 3, dans lequel le solvant organique miscible à l'eau est :
(i) un alcool, une cétone aliphatique, de l'acétonitrile, du formamide, du N,N-diméthylformamide, du diméthylsulfoxyde, du sulfolane, du diglyme, du dioxane, du tétrahydrofurane ou de la N-méthylpyrrolidine ; et/ou
(ii) un alcool alkylique inférieur ; et/ou
(iii) du méthanol.

5. Procédé selon l'une quelconque des revendications 1 à 4, dans lequel :
(i) à l'étape (a), la solution aqueuse d'un sel de calcium et la solution d'un sel de zofénopril dans un solvant organique miscible à l'eau sont mélangées selon un rapport eau/solvant organique de 1/1 à 1/10 ; et/ou
(ii) le mélange de l'étape (a) est effectué à une température pouvant atteindre 85 °C ; et/ou
(iii) la conservation de l'étape (b) est effectuée sous agitation ; et/ou
(iv) la conservation de l'étape (b) est effectuée pendant au moins une heure ; et/ou
(v) la conservation de l'étape (b) est effectuée à une température pouvant atteindre 85 °C ; et/ou
(vi) le procédé est effectué sans ensemencement du zofénopril calcium de forme A ; et/ou
(vii) la séparation de l'étape (c) est effectuée en filtrant la suspension ; et/ou
(viii) la séparation de l'étape (c) est effectuée à une température pouvant atteindre 85 °C ; et/ou
(ix) le zofénopril calcium séparé est purifié par rinçage à l'eau ou au méthanol ; et/ou
(x) le zofénopril calcium séparé est purifié par rinçage au méthanol par agitation du zofénopril calcium dans du méthanol et filtration ; et/ou
(xi) le zofénopril calcium séparé est séché sous pression réduite à une température pouvant atteindre 85 °C.

6. Procédé selon l'une quelconque des revendications 1 à 5, dans lequel le zofénopril calcium préparé est le zofénopril calcium de forme A.

7. Procédé selon la revendication 6, dans lequel le zofénopril calcium de forme A est sensiblement exempt d'autres formes de zofénopril calcium.

8. Procédé selon la revendication 6 ou 7, dans lequel le zofénopril calcium de forme A présente des pics XRD sensiblement aux angles de diffusion 2θ suivants : 4,3, 7,4, 8,7, 10,1, 10,8, 11,7, 13,0, 14,8, 16,0, 17,2, 18,2, 19,0, 20,0, 21,7, 23,5 et 24,6.

9. Procédé selon l'une quelconque des revendications 6 à 8, dans lequel le zofénopril calcium de forme A comprend moins de 6 % d'autres formes de zofénopril calcium.

10. Procédé selon l'une quelconque des revendications 1 à 9, dans lequel le zofénopril calcium préparé a une pureté chimique supérieure à 98,5 %.
